# EUROPEAN PATENT APPLICATION

(11) **EP 4 289 809 A1**
(43) Date of publication of application: **13.12.2023**
(21) Application number: 22749820.1
(22) Date of filing: 04.02.2022
(51) Int. Cl.: C07C 41/08, C07C 41/42, C07C 41/44, C07C 43/16, C07B 61/00

(54) **METHOD FOR PRODUCING ISOBUTYL VINYL ETHER AND METHOD FOR PURIFYING ISOBUTYL VINYL ETHER**

(30) Priority: 04.02.2021 JP 2021016615
(71) Applicant: Maruzen Petrochemical Co., Ltd., Tokyo 104-8502 (JP)
(72) Inventor: ITO, Yudai, Ichihara-shi, Chiba 290-8503 (JP); SUDO, Masahiro, Ichihara-shi, Chiba 290-8503 (JP); NANIKI, Takashi, Ichihara-shi, Chiba 290-8503 (JP)
(74) Representative: Wächtershäuser & Hartz Patentanwaltspartnerschaft mbB
(86) International application number: PCT/JP2022/004489
(87) International publication number: WO 2022/168949

(57) **Abstract**

Provided is a method for efficiently producing isobutyl vinyl ether with a high purity from isobutyl alcohol and acetylene.

The present invention is a method for producing isobutyl vinyl ether using isobutyl alcohol as a raw material alcohol, the method includes the following steps A1, A2, and A3:
(Step A1) a vinyl ether synthesis step of causing isobutyl alcohol to react with acetylene to obtain a mixture containing unreacted isobutyl alcohol and isobutyl vinyl ether;
(Step A2) an acetalization step of causing a reaction of the unreacted isobutyl alcohol and the isobutyl vinyl ether in the mixture in the presence of an acid catalyst to be converted into acetaldehyde diisobutyl acetal; and
(Step A3) a distillation step of removing the acetaldehyde diisobutyl acetal by distillation from an acetal-containing mixture obtained in step A2.

## Description

### Technical Field

The present invention relates to a method for producing isobutyl vinyl ether and a method for purifying isobutyl vinyl ether.

### Background Art

As a method for producing vinyl ether, an addition reaction of an alcohol to acetylene is widely known. This method makes it difficult to purify the vinyl ether by distillation when a raw material alcohol remaining in a resulting crude vinyl ether forms an azeotropic mixture with a target vinyl ether.

As an alcohol forming the azeotropic mixture and vinyl ether corresponding to the alcohol, for example, a combination of 2-ethylhexanol and 2-ethylhexyl vinyl ether and a combination of cyclohexanol and cyclohexyl vinyl ether are known. Accordingly, a method has been proposed that causes unreacted 2-ethylhexanol or cyclohexanol contained in a mixture obtained by an addition reaction of 2-ethylhexanol or cyclohexanol to acetylene to react with 2-ethylhexyl vinyl ether or cyclohexyl vinyl ether produced by the addition reaction described above in the presence of an acid catalyst to be converted into acetal and then remove the acetal (Patent Literature 1).

### Citation List

### Patent Literature

Patent Literature 1: JP 5312133 B2

### Summary of the Invention

### Problem to be solved by the Invention

However, there are few reports on production of isobutyl vinyl ether from isobutyl alcohol and acetylene. Further, it has not been known heretofore that a combination of isobutyl alcohol and isobutyl vinyl ether forms an azeotropic mixture.

Under such a background, the present inventors conducted study. As a result of that, they found that a combination of isobutyl alcohol and isobutyl vinyl ether forms the azeotropic mixture.

The present invention provides a method for efficiently producing isobutyl vinyl ether with a high purity from isobutyl alcohol and acetylene.

### Means of Solving the Problem

The problem according to the present invention has been solved by the following means <1> to <6>.
<1> A method for producing isobutyl vinyl ether using isobutyl alcohol as a raw material alcohol, the method including the following steps A1, A2, and A3:
   (Step A1) a vinyl ether synthesis step of causing isobutyl alcohol to react with acetylene to obtain a mixture containing unreacted isobutyl alcohol and isobutyl vinyl ether;
   (Step A2) an acetalization step of causing a reaction of the unreacted isobutyl alcohol and the isobutyl vinyl ether in the mixture in the presence of an acid catalyst to be converted into acetaldehyde diisobutyl acetal; and
   (Step A3) a distillation step of removing the acetaldehyde diisobutyl acetal by distillation from an acetal-containing mixture obtained in step A2.
<2> A method for producing isobutyl vinyl ether using isobutyl alcohol as a raw material alcohol, the method including the following steps A1, A2, A2-2, and A3:
   (Step A1) a vinyl ether synthesis step of causing isobutyl alcohol to react with acetylene to obtain a mixture containing unreacted isobutyl alcohol and isobutyl vinyl ether;
   (Step A2) an acetalization step of causing a reaction of the unreacted isobutyl alcohol and the isobutyl vinyl ether in the mixture in the presence of an acid catalyst to be converted into acetaldehyde diisobutyl acetal; and
   (Step A2-2) a step of performing a treatment selected from the group consisting of neutralization of the acid catalyst and removal of the acid catalyst; and
   (Step A3) a distillation step of removing the acetaldehyde diisobutyl acetal by distillation from an acetal-containing mixture obtained in Step A2-2.
<3> The production method according to <2>, wherein step A2-2 is a step of performing a neutralization treatment of the acid catalyst using a basic compound.
<4> The production method according to any one of <1> to <3>, wherein step A1 is performed in the presence of an alkali metal alcoholate catalyst.
<5> The production method according to any one of <1> to <4>, wherein a distillation pressure in step A3 is 40 kPaA to atmospheric pressure.
<6> A method for purifying isobutyl vinyl ether from a mixture containing isobutyl alcohol and isobutyl vinyl ether, the method including the following steps B1 and B2:
   (Step B1) an acetalization step of causing a reaction of isobutyl alcohol and isobutyl vinyl ether in the mixture in the presence of an acid catalyst to be converted into acetaldehyde diisobutyl acetal; and
   (Step B2) a distillation step of removing the acetaldehyde diisobutyl acetal by distillation from an acetal-containing mixture obtained in step B1.

### Effects of the Invention

According to the production method of the present invention, isobutyl vinyl ether can be efficiently produced with a high purity from isobutyl alcohol and acetylene.

Further, according to the purification method of the present invention, isobutyl vinyl ether can be efficiently purified with a high purity from a mixture containing isobutyl alcohol and isobutyl vinyl ether.

### Brief Description of Drawings

Fig. 1 is a gas-liquid equilibrium diagram of isobutyl vinyl ether and isobutyl alcohol at 80 kPaA and 15 kPaA.
Fig. 2 is a schematic diagram illustrating a configuration of an apparatus used in examples.

### Detailed Description of the Invention

### [Method for producing vinyl ether]

The production method of the present invention is a method for producing isobutyl vinyl ether using isobutyl alcohol as a raw material alcohol, and includes the steps A1, A2, and A3. Specifically, after step A1 (vinyl ether synthesis step) is performed, step A1-2 (catalyst removal step) is performed, if necessary, on a mixture containing an unreacted raw material alcohol and isobutyl vinyl ether obtained in the step (mixture further containing a catalyst when a vinyl ether synthesis is performed in the presence of a catalyst), and then step A3 (distillation step) is performed after step A2 (acetalization step).

### (Step A1 Vinyl ether synthesis step)

Step A1 is a vinyl ether synthesis step of causing isobutyl alcohol (boiling point: 108°C) to react with acetylene to obtain a mixture containing an unreacted raw material alcohol and isobutyl vinyl ether (boiling point: 83°C). Isobutyl vinyl ether is a vinyl ether with a low boiling point, and the production method of the present invention can produce such isobutyl vinyl ether with a high purity, being a vinyl ether with a low boiling point.

Step A1 is preferably performed in the presence of a catalyst. As the catalyst, an alkali metal alcoholate catalyst is preferable from the viewpoint of reaction efficiency.

The alkali metal alcoholate catalyst is an alkali metal alcoholate of an alkali metal hydroxide and isobutyl alcohol, and is preferably dissolved in isobutyl alcohol from the viewpoint of handleability. Specific examples of the alkali metal hydroxide include sodium hydroxide, potassium hydroxide, rubidium hydroxide, and cesium hydroxide, and one of these may be used alone, or two or more kinds thereof may be used in combination.

In step A1, an organic solvent may be used, and as the organic solvent, for example, an aprotic polar solvent that is mixed with isobutyl alcohol and dissolves an alkali metal alcoholate catalyst is preferable. Examples thereof include amide solvents such as dimethylacetamide, 2-pyrrolidone, N-methyl-2-pyrrolidone, and 1,3-dimethyl-2-imidazolidinone; sulfur-containing compound solvents such as sulfolane and dimethyl sulfoxide; and glycol dialkyl ether solvents such as diethylene glycol dimethyl ether, diethylene glycol diethyl ether, triethylene glycol dimethyl ether, triethylene glycol diethyl ether, tetraethylene glycol dimethyl ether, and tetraethylene glycol diethyl ether. One of these solvents may be used alone, or two or more kinds thereof may be used in combination.

A reaction temperature in step A1 is usually in a range of 60 to 200°C, and more preferably in a range of 80 to 150°C from the viewpoint of reaction rate and side reaction restraint. The reaction rate increases as a reaction pressure increases, but it is preferable to set the reaction pressure to 0.3 MPa or less in order to prevent decomposition explosion of acetylene. Further, the reaction time of step A1 is usually about 10 minutes to 48 hours.

### (Step A1-2 Catalyst removal step)

In the production method of the present invention, when the vinyl ether synthesis is performed in the presence of a catalyst, the catalyst, for example, may be removed from a reaction mixture obtained in step A1 prior to step A2.

Removal of the catalyst, for example, can be performed by a known method such as solid-liquid separation (in a case of a solid catalyst or a supported catalyst) such as solvent extraction, distillation, and filtration. Among these methods, a method by distillation is preferable in that the catalyst can be easily separated and the raw material alcohol can be reduced in advance. Further, in a case of the method by distillation, the organic solvent at the time of performing step A1 using an organic solvent can also be removed. Further, when the catalyst is removed by a method other than distillation, further distillation may be performed to reduce the raw material alcohol in the reaction mixture.

A distillation column used for removal of the catalyst, for example, or distillation for concentration after removal of the catalyst (hereinafter, also referred to as "preliminary distillation") may be any one of a packed column, a plate column, a bubble cap column, for example, and number of plates of the distillation column is, for example, 1 to 100, and preferably 5 to 50 in term of theoretical plates.

The preliminary distillation may be performed under any condition of normal pressure, increased pressure, and reduced pressure, and is preferably performed under normal pressure or reduced pressure. Specifically, the pressure is usually 0.7 to 13.3 kPa, and preferably 1.3 to 6.7 kPa. Further, a distillation method may be any of a batch method, a semi-batch method, or a continuous method.

### (Step A2 Acetalization step)

Step A2 is an acetalization step of causing a reaction of the unreacted raw material alcohol with the isobutyl vinyl ether in the mixture obtained in step A1 or step A1-2 in the presence of an acid catalyst to be converted into acetaldehyde diisobutyl acetal.

As described in examples to be described later, it has been found that a combination of isobutyl alcohol as a raw material alcohol and isobutyl vinyl ether forms an azeotropic mixture, but by performing step A2, the unreacted raw material alcohol is subjected to acetalization, and a purity of the isobutyl vinyl ether can be easily increased by a distillation operation, despite the fact that a raw material alcohol and a target vinyl ether form the azeotropic mixture in this manner.

Examples of the acid catalyst used in step A2 include inorganic acids such as sulfuric acid, nitric acid, hydrochloric acid, and phosphoric acid; organic acids such as carboxylic acid and organic sulfonic acid; and solid acid catalysts such as acidic zeolite, heteropoly acid, and a strong acid ion□exchange resin. One of these solvents may be used alone, or two or more kinds thereof may be used in combination.

Among them, from the viewpoint of side reaction restraint (particularly, restraint of polymerization reaction of vinyl ether), phosphoric acid, organic sulfonic acid, and a sulfonic acid type strong acid ion-exchange resin (a strong acid ion-exchange resin having a sulfonic acid group in the molecule) are preferable, and organic sulfonic acid is more preferable.

Examples of the organic sulfonic acid include aromatic sulfonic acids such as p-toluene sulfonic acid, o-toluene sulfonic acid, benzenesulfonic acid, p-xylene-2-sulfonic acid, dodecylbenzene sulfonic acid, 1-naphthalene sulfonic acid, 2-naphthalene sulfonic acid, dinonylnaphthalene sulfonic acid, and dinonylnaphthalene disulfonic acid; aliphatic sulfonic acids such as methane sulfonic acid, ethane sulfonic acid, and trifluoromethanesulfonic acid; and aromatic sulfonates such as pyridinium p-toluene sulfonate and quinolinium p-toluene sulfonate.

Examples of the strong acid ion-exchange resin include a sulfonic acid type strong acid ion-exchange resin and a mixture of a sulfonic acid type strong acid ion-exchange resin and an amine type weakly basic ion-exchange resin. Examples of commercially available products of the sulfonic acid type strong acid ion-exchange resin include Amberlyst 15DRY manufactured by Organo Corporation, and examples of commercially available products of a mixture of the sulfonic acid type strong acid ion-exchange resin and the amine type weakly basic ion-exchange resin include Amberlyst MSPS 2-1 · DRY manufactured by Organo Corporation.

From the viewpoint of the purity of isobutyl vinyl ether, the amount of the acid catalyst used is usually 0.00001 to 5 parts by mass, preferably 0.0001 to 1 part by mass, more preferably 0.001 to 0.1 part by mass, and still more preferably 0.001 to 0.01 part by mass, based on 100 parts by mass of isobutyl vinyl ether.

A method for adding an acid catalyst to the mixture obtained in step A1 or step A1-2 may be appropriately selected according to a type of the acid catalyst. For example, in a case of an inorganic acid or an organic acid, the acid may be added to the mixture obtained in step A1 or step A1-2 as it is or after being dissolved in an appropriate solvent (preferably, isobutyl alcohol). Further, in a case of solid acid catalyst, the solid acid catalyst may be directly added to the mixture obtained in step A1 or step A1-2, or the solid acid catalyst may be packed in a column container, for example, and the mixture obtained in step A1 or step A1-2 may be passed through the column container, for example.

The reaction temperature in step A2 is preferably in a range of 0 to 80°C, more preferably in a range of 10 to 60°C, from the viewpoint of reaction rate and side reaction restraint.

The reaction time of step A2 is usually about 10 minutes to 48 hours.

The acetal obtained in step A2 is acetaldehyde diisobutyl acetal (boiling point: 171°C).

Further, the production method of the present invention preferably includes a step of performing a treatment selected from the group consisting of neutralization of the acid catalyst and removal of the acid catalyst (step A2-2) after step A2 and prior to step A3, in order to suppress production of a heavy material in step A3.

Further, when an inorganic acid or an organic acid is used as the acid catalyst, neutralization of the acid catalyst is preferable as step A2-2. The neutralization of the acid catalyst may be performed using a basic compound. Examples of the basic compound include alkali metal compounds such as alkali metal hydroxides (for example, sodium hydroxide and potassium hydroxide), alkali metal carbonates, and alkali metal hydrogen carbonates; and a basic ion-exchange resin. Further, examples of the alkali metal include sodium and potassium.

From the viewpoint of the purity of isobutyl vinyl ether, the amount of the basic compound used is usually 1 to 1000 molar equivalents, preferably 5 to 100 molar equivalents, more preferably 10 to 100 molar equivalents, and particularly preferably 20 to 100 molar equivalents, relative to the acid catalyst used in step A2. When the amount of the basic compound used is 20 molar equivalents or more, isobutyl vinyl ether is obtained particularly efficiently.

When an alkali metal compound is used as the basic compound, the alkali metal compound may be added to an acetal-containing mixture obtained in step A2 as it is or after being dissolved in an appropriate solvent (preferably water). Further, when the basic ion-exchange resin is used, the basic ion-exchange resin may be directly added to the acetal-containing mixture obtained in step A2, or the basic ion-exchange resin may be packed in a column container, and the acetal-containing mixture obtained in step A2 may be passed through the column container. Further, when a solid or a precipitate is present in a liquid after neutralization, the solid or the precipitate may be subjected to solid-liquid separation by filtration or centrifugation as necessary.

On the other hand, when, for example, a solid acid catalyst is used as the acid catalyst, removal of the acid catalyst is preferable as step A2-2. Examples of a removal operation include solid-liquid separation operations such as filtration and centrifugation. Note that, when the solid acid catalyst is packed in a column container and used, a separation operation is unnecessary.

### (Step A3 Distillation step)

Step A3 is a distillation step of removing the acetaldehyde diisobutyl acetal by distillation from the acetal-containing mixture obtained in step A2 (step A2-2 when step A2-2 is performed).

A distillation apparatus and distillation method to be used in step A3 are not particularly limited, and number of distillation plates may be provided even in a simple distillation. The distillation method may be any of a batch method, a semi-batch method, and a continuous method. Further, when a distillation column is used, the distillation column may be, for example, any of a packed column, a plate column, and a bubble cap column. The number of plates of the distillation column is preferably 1 to 30 plates, more preferably 5 to 15 plates in terms of theoretical plates. Further, a reflux ratio is preferably in a range of 1 to 15.

Further, an overhead temperature of the distillation column is preferably 40 to 100°C and more preferably 50 to 90°C, and a bottom temperature of the distillation column is preferably 40 to 180°C and more preferably 50 to 170°C.

A distillation pressure in step A3 is preferably 20 to 120 kPaA (where A represents an absolute pressure), more preferably 40 kPaA to atmospheric pressure from the viewpoint of the purity of isobutyl vinyl ether.

When a distillation column is used, the target isobutyl vinyl ether with a high purity is obtained from an overhead of the distillation column, and a bottom liquid rich in acetaldehyde diisobutyl acetal is recovered from a bottom of the column. The acetaldehyde diisobutyl acetal contained in the bottom liquid can be recovered, converted into vinyl ether and a raw material alcohol, and recycled as a raw material for isobutyl vinyl ether synthesis.

### (Step A4 Acetal decomposition step)

As a method for converting acetaldehyde diisobutyl acetal into a raw material alcohol and vinyl ether, a known method may be appropriately used. Specific examples thereof include a method of thermally decomposing in a gas phase in the presence of a silica/alumina-based catalyst carrying an alkali or alkaline earth metal (for example, Khim. Prom.48(9)657-660(1972), JP 48-78109 A, JP 62-87247 A, for example), a method of decomposing in a gas phase using magnesium oxide as a catalyst (JP H8-268945 A), a method of decomposing in the presence of a catalyst containing a noble metal (for example, Ann.,601 81-84,1956, DE 1957680 A1, JP 48-76803 A, for example), and a method of decomposing using an acid catalyst (for example, J.Org.Chem.,38,2910,1973, Helv.Chim.Acta,1158 (1967), Bull.Chem.Soc.Jpn.3089(1976), JP H8-277237 A, for example).

### [Method for purifying isobutyl vinyl ether]

The purification method of the present invention is a method for purifying isobutyl vinyl ether from a mixture containing isobutyl alcohol and isobutyl vinyl ether, and includes the following steps B1 and B2:
(Step B1) an acetalization step of causing a reaction of isobutyl alcohol and isobutyl vinyl ether in the mixture in the presence of an acid catalyst to be converted into acetaldehyde diisobutyl acetal; and
(Step B2) a distillation step of removing the acetaldehyde diisobutyl acetal by distillation from an acetal-containing mixture obtained in step B1.

Step B1 may be performed in the same manner as step A2 in the production method of the present invention, and step B2 may be performed in the same manner as step A3 in the production method of the present invention.

### Examples

Hereinafter, the present invention will be described in detail with reference to examples. But, the present invention is not limited to these examples.

### [Reference Example 1 Gas-liquid equilibrium of isobutyl vinyl ether and isobutyl alcohol (80 kPaA)]

Isobutyl vinyl ether and isobutyl alcohol were charged into an Othmer equilibrium distillation apparatus, the pressure was adjusted to 80 kPaA, and then heating was started. After reaching an equilibrium state from the start of reflux, a vapor phase condensate and a liquid phase were sampled and subjected to a composition analysis.

Next, isobutyl alcohol was added into the system, and the same procedure was performed.

Thereafter, the addition of isobutyl alcohol into the system was repeated, and a gas-liquid equilibrium diagram of isobutyl vinyl ether and isobutyl alcohol at 80 kPaA was prepared.

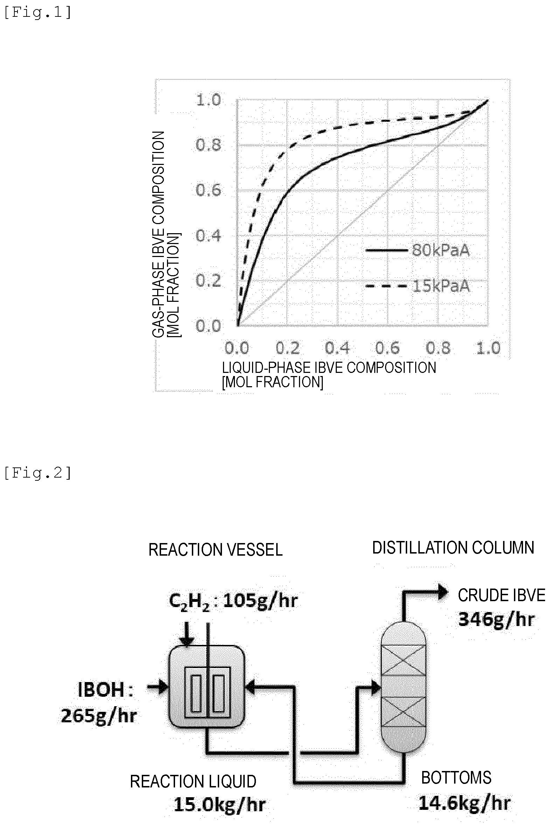

Fig. 1 shows a gas-liquid equilibrium diagram of isobutyl vinyl ether and isobutyl alcohol at 80 kPaA. Note that IBVE in Fig. 1 means isobutyl vinyl ether.

A composition at an intersection point of a gas-liquid equilibrium line and a diagonal line, that is, at an azeotropic point was 96 mass% of isobutyl vinyl ether and 4 mass% of isobutyl alcohol, and the temperature at that time was 76°C.

### [Reference Example 2 Gas-liquid equilibrium of isobutyl vinyl ether and isobutyl alcohol (15 kPaA)]

The same operation as in Reference Example 1 was performed except that the pressure was changed to 15 kPaA.

The composition at the azeotropic point was 97 mass% of isobutyl vinyl ether and 3 mass% of isobutyl alcohol, and the temperature at that time was 32°C. The results are shown in Fig. 1.

From the gas-liquid equilibrium diagram shown in Fig. 1, it found that isobutyl vinyl ether is azeotropic with isobutyl alcohol as a raw material, so that general distillation purification is difficult.

### [Example 1 Production of isobutyl vinyl ether (IBVE) (1)]

### (Vinyl etherification step and catalyst removal step)

A stainless steel autoclave with a volume of 10 L was used as a reaction vessel, and a stainless steel packed type continuous distillation column (packing: Sumitomo Heavy Industries, Ltd., Sumitomo/Sulzer Laboratory packing) with an inner diameter of 50 mmϕ, 12 theoretical plates (with 7 supply plates) and a pot volume of 4 L was used as a continuous distillation column. Fig. 2 shows a schematic diagram illustrating the configuration of the apparatus used in the reaction.

An autoclave was filled with 6.14 kg of isobutyl alcohol (IBOH), 0.90 kg of potassium hydroxide, and 0.56 kg of tetraglyme. An alcoholate catalyst (a potassium-isobutyl alcoholate) was prepared for 15 hours under autoclave conditions of 100°C, 0.03 MPaG, and a maximum retention oil amount of 5 L, and continuous distillation column conditions: an overhead temperature of 65 to 68°C, a bottom temperature of 100 to 120°C, 17 to 43 kPa, a maximum retention oil amount of 2 L, a reflux ratio of 8, and a circulation flow rate between the autoclave and the distillation column of 15 kg/hr. The amount of distillate from the overhead of the continuous distillation column during this period was 2.04 kg, containing 0.28 kg of water.

After 0.50 kg of isobutyl alcohol and 1.46 kg of tetraglyme were additionally filled, the autoclave conditions were changed to 110°C and 0.06 MPaG, and acetylene and isobutyl alcohol were continuously supplied at a rate of 105 g/hr and 265 g/hr, respectively.

Next, a reaction liquid obtained in the above reaction was continuously supplied to the continuous distillation column at a rate of 15.0 kg/hr. Note that the composition of this reaction liquid was 38.4 mass% of isobutyl alcohol, 8.5 mass% of isobutyl vinyl ether, 25.7 mass% of potassium-isobutyl alcoholate, 27.1 mass% of tetraglyme, and 0.3 mass% of a heavy component.

Further, under the conditions of a reflux ratio of 3 to 4, an overhead pressure of 60 kPaA, an overhead temperature of 65°C, and a bottoms temperature of 120 to 125°C, bottoms was extracted from the bottom of the continuous distillation column at 14.6 kg/hr and supplied to the autoclave. Note that the composition of the bottoms from the continuous distillation column was 39.1 mass% of isobutyl alcohol, 6.4 mass% of isobutyl vinyl ether, 26.4 mass% of potassium-isobutyl alcoholate, 27.8 mass% of tetraglyme, and 0.3 mass% of a heavy component.

In this way, a crude vinyl ether was continuously synthesized, and the crude vinyl ether was obtained at a flow rate of 346 g/hr from the overhead of the continuous distillation column. Note that the composition of the crude vinyl ether was 95.3 mass% of isobutyl vinyl ether and 4.7 mass% of isobutyl alcohol.

### (Acetalization reaction step and distillation purification step)

679.6 g of A crude IBVE obtained in the above step was weighed into a 1000 mL three-necked flask equipped with a stirrer chip, 1.04 g of an IBOH solution of p-toluene sulfonic acid (p-toluene sulfonic acid concentration: 2 mass%, p-toluene sulfonic acid at 30 ppm relative to the crude IBVE) was added thereto, and the mixture was stirred in a water bath set at 25°C for 60 minutes. IBOH in the solution after reaction was 0.1 mass% or less, and IBVE and acetaldehyde diisobutyl acetal were contained in an amount of 89 mass% and 11 mass%, respectively. After neutralization by adding 0.36 g of a potassium hydroxide aqueous solution (potassium hydroxide concentration: 42.1 mass%, potassium hydroxide at 210 ppm relative to a reaction solution, 22 molar equivalents relative to p-toluene sulfonic acid) to the reaction solution, distillation was performed using a packed column having 10 theoretical plates (internal pressure: 80 kPaA, column bottom setting temperature: 80 to 160°C, and refrigerant setting temperature: 5°C) to obtain 554.8 g of a high-purity IBVE having a purity of 99 mass% or more (a recovery rate based on IBVE: 85.7 mass%).

### [Example 2 Production of IBVE (2)]

### (Acetalization reaction step and distillation purification step)

A crude IBVE was obtained by performing the vinyl etherification step and the catalyst removal step in the same manner as in Example 1, then 791.4 g of the resulting crude IBVE was weighed into a 2000-mL three-necked flask equipped with a stirrer chip, 1.2 g of an IBOH solution of p-toluene sulfonic acid (p-toluene sulfonic acid concentration: 2 mass%, p-toluene sulfonic acid at 30 ppm relative to the crude IBVE) was added thereto, and the mixture was stirred in a water bath set at 25°C for 60 minutes. IBOH in the solution after reaction was 0.1 mass% or less, and IBVE and acetaldehyde diisobutyl acetal were contained in an amount of 89 mass% and 11 mass%, respectively. After neutralization by adding 0.08 g of an aqueous sodium hydroxide solution (sodium hydroxide concentration: 34.3 mass%, sodium hydroxide at 35 ppm relative to the reaction solution, 5 molar equivalents relative to p-toluene sulfonic acid) to the reaction solution, distillation was performed using a packed column having 10 theoretical plates (internal pressure: 80 kPaA, column bottom setting temperature: 80 to 160°C, and refrigerant setting temperature: 5°C) to obtain 557.2 g of a high-purity IBVE having a purity of 99 mass% or more (the recovery rate based on IBVE: 73.7 mass%).

## Claims

1. A method for producing isobutyl vinyl ether using isobutyl alcohol as a raw material alcohol, the method comprising the following steps A1, A2, and A3:
(Step A1) a vinyl ether synthesis step of causing isobutyl alcohol to react with acetylene to obtain a mixture containing unreacted isobutyl alcohol and isobutyl vinyl ether;
(Step A2) an acetalization step of causing a reaction of the unreacted isobutyl alcohol and the isobutyl vinyl ether in the mixture in the presence of an acid catalyst to be converted into acetaldehyde diisobutyl acetal; and
(Step A3) a distillation step of removing the acetaldehyde diisobutyl acetal by distillation from an acetal-containing mixture obtained in step A2.

2. A method for producing isobutyl vinyl ether using isobutyl alcohol as a raw material alcohol, the method comprising the following steps A1, A2, A2-2, and A3:
(Step A1) a vinyl ether synthesis step of causing isobutyl alcohol to react with acetylene to obtain a mixture containing unreacted isobutyl alcohol and isobutyl vinyl ether;
(Step A2) an acetalization step of causing a reaction of the unreacted isobutyl alcohol and the isobutyl vinyl ether in the mixture in the presence of an acid catalyst to be converted into acetaldehyde diisobutyl acetal;
(Step A2-2) a step of performing a treatment selected from the group consisting of neutralization of the acid catalyst and removal of the acid catalyst; and
(Step A3) a distillation step of removing the acetaldehyde diisobutyl acetal by distillation from an acetal-containing mixture obtained in step A2-2.

3. The production method according to Claim 2, wherein step A2-2 is a step of performing a neutralization treatment of the acid catalyst using a basic compound.

4. The production method according to any one of Claims 1 to 3, wherein step A1 is performed in the presence of an alkali metal alcoholate catalyst.

5. The production method according to any one of Claims 1 to 4, wherein a distillation pressure in step A3 is 40 kPaA to atmospheric pressure.

6. A method for purifying isobutyl vinyl ether from a mixture containing isobutyl alcohol and isobutyl vinyl ether, the method comprising the following steps B1 and B2:
(Step B1) an acetalization step of causing a reaction of isobutyl alcohol and isobutyl vinyl ether in the mixture in the presence of an acid catalyst to be converted into acetaldehyde diisobutyl acetal; and
(Step B2) a distillation step of removing the acetaldehyde diisobutyl acetal by distillation from an acetal-containing mixture obtained in step B1.
